(19) **Europäisches Patentamt / European Patent Office / Office européen des brevets**

(11) **EP 4 017 643 B1**

(12) **EUROPEAN PATENT SPECIFICATION**

(45) Date of publication and mention
of the grant of the patent:
**19.11.2025 Bulletin 2025/47**

(21) Application number: **20765109.2**

(22) Date of filing: **12.08.2020**

(51) International Patent Classification (IPC):
**B04B 5/02** (2006.01)   **B04B 13/00** (2006.01)

(52) Cooperative Patent Classification (CPC):
**B04B 5/02; B04B 13/00**

(86) International application number:
**PCT/SE2020/050779**

(87) International publication number:
**WO 2021/034249 (25.02.2021 Gazette 2021/08)**

(54) **SEPARATING PARTICLES THROUGH CENTRIFUGAL SEDIMENTATION**

ABSCHEIDUNG VON PARTIKELN DURCH ZENTRIFUGALSEDIMENTATION

SÉPARATION DE PARTICULES PAR SÉDIMENTATION CENTRIFUGE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.08.2019 SE 1950957**

(43) Date of publication of application:
**29.06.2022 Bulletin 2022/26**

(73) Proprietor: **Grimaldi Development AB
131 26 Nacka Strand (SE)**

(72) Inventors:
- **INGE, Claes
  131 72 Nacka (SE)**
- **FRANZÉN, Peter
  124 76 Bandhagen (SE)**
- **HÄGGMARK, Carl Petrus
  187 51 Täby (SE)**

(74) Representative: **Fenix Legal KB
Brahegatan 44
114 37 Stockholm (SE)**

(56) References cited:
**WO-A1-2011/081531    WO-A1-2019/091650
WO-A1-2019/091880**

## Description

## Field of the invention

This invention relates to a method of separating particles having different sedimentation velocities in a fluid sample through centrifugal sedimentation. The invention also relates to an apparatus for performing the method.

## Background of the invention

When curing blood poisoning, for example, the pathogenic bacteria have to be detected among blood cells. The bacteria then usually have to be cultivated from a blood draw. Occasionally, the cultivation process may however take such a long time that the patient risks dying of blood poisoning before the bacteria are identified.

WO 2019/091880 A1 discloses a method and apparatus for controlling a focus point of a stationary beam focusing on a sample in a rotating cartridge placed in a rotating disc. Therein are used systems known from WO 2019/091650 A1 and WO 2011/081531 A1, each disclosing a dual axis centrifugation apparatus for a sample processing device. A cartridge which is a processing device for a sample is arranged in holding means in a device rotating around its own axis. This device is installed in a bigger rotating device or disc. By means of mechanical transmission devices, controllers and motors, rotation speed of each rotating device is individually controlled. The cartridge can then be rotated around its own axis as well as around a distal axis. The rotation around the distal axis is for orienting the sample into different channels and microfluidic channels during a centrifugation process.

## Disclosure of the invention

An object of this invention is to develop a method where particles such as bacteria, in a fluid sample such as blood, can be separated from other particles such as blood cells in a new and time saving centrifugal manner.

In the invention the method comprises:

    a) enclosing the sample; and
    b) rotating the sample about a primary axis outside the sample at a first rotational speed and about a secondary axis located in a center of the sample at a second rotational speed, for subjecting the sample to a varying centrifugal field until each particle has settled at a position which depends on the sedimentation velocity of the particle.

As the sample is thereby subjected to rotation about two different axes, a particle will sometimes be between the primary axis and the centre axis of the cylinder and sometimes be located radially outside both axes. This causes the particle to alternately move from and towards the axis of the cylinder. Thereby, the sedimentation of particles of a certain size will at some point be counteracted by the rotation of the secondary axis so that the particle lies still relative to the primary axis. Each particle size has its equilibrium position when settled in the sample. Calculations show that the particle will search for the equilibrium position regardless of where in the sample it starts. The equilibrium position depends, among other things, on the particle's sedimentation velocity, which in turn is affected by the size. The larger the particle, the farther from the secondary axis it ends up, and supposedly always on a straight line from the secondary axis.

While not necessarily required for performing the method, the first and second axes are conveniently parallel to each other.

An insight that underlies the invention is that there should be possible to separate particles such as bacteria from blood cells provided that there is a small difference in sedimentation rate. That is, it should be possible to fractionate particles of different sizes. A particle of a certain size will then search for equilibrium and it is possible to calculate how long it takes.

Another application is to produce monodisperse particles, i.e. particles or particle clusters where all particles have the same size. Such particles are used inter alia to calibrate particle analyzers that analyze the frequency of particles having a certain size. Still other applications are imaginable.

In another embodiment of the invention the equilibrium or end position of particles as calculated or obtained by previous operations of the method may then be used to more easily find or spot the particles of interest to be further analysed and eventually identified. The method may then further comprise:

    c) spotting the particles in the settled fractions in the sample;
    d) measuring a distance of each particle from the secondary axis; and
    e) identifying the particles by matching said distance with distances obtained by calculation for known particles theoretically subjected to the same method.

Alternatively, in yet another embodiment the method may comprise:

    c) spotting the particles in the settled fractions in the sample;
    d) measuring a distance of each particle from the secondary axis;
    f) identifying the particles by matching said distance with distances obtained by performing the above steps a-d for known particles.

For ease of identification, steps e and f may then comprise tabulated data for sets of known particles in areas of interest.

The step of measuring the distance is here considered inherently equivalent to determining the sedimentation rate/velocity under the given conditions.

If the separator is stopped a flow will be initiated in the cylindrical container. This flow will mix the fractionated particles. To avoid this, spotting or finding of the particles in the settled fractions may be facilitated by blocking different regions of the sample from mixing with each other.

Specifically, the blocking may be obtained by inserting a framework or grid of axially open compartments into the sample having the settled fractions, for dividing the sample into said regions defined by the compartments. A radially succession of compartments may then function as a multistage filter that facilitates identification of particles found therein. Particles found in a certain compartment may then be more easily identified by being in a close range of sedimentation rates or end locations for known particles of interest.

In the bacteria application the purpose is to concentrate the bacteria level and lessen the blood cell level that may interfere with subsequent analysis. After the separation process the type of bacteria is analyzed by other methods.

It would also be possible to sample particles from different regions in separate samples.

The second rotational speed is higher than the first rotational speed. In contrast, the relative rotational speed, $\omega$, of the sample should be very small in relation to the main rotational speed, $\Omega$, i.e. first rotational speed. As further explained later, $\omega$ may however not be too small not to prolong the separation process.

The sample may be a blood sample. In that application bacteria can be separated from blood cell as mentioned above.

An apparatus for performing the method of the invention comprises a cylindrical container for enclosing the sample;

> a first rotator for rotating the container about the primary axis; and
> a second rotator for rotating the container about the secondary axis;
> wherein the first rotator comprises a disk supported for rotation about the primary axis and a motor for rotating the disk, and the second rotator comprises a gear or belt transmission for rotating the cylindrical container supported at the disk about the secondary axis by the motor.

In an alternative embodiment the second rotator comprises a stationary gear concentric with the primary axis and in gear engagement with a gear for rotating the cylindrical container supported at the disk for rotation about the secondary axis. Importantly, such arrangement will make certain that the first and second rotational speeds are synchronized. Other arrangements, e.g. belt arrangements, may be equally practicable.

The apparatus in another embodiment may also comprise a plurality of said container peripherally distributed around the primary axis. Thereby it will be possible to process larger volumes of samples in a single separation process.

Other features and advantages of the invention may be apparent in the following detailed description and the appended claims.

## Brief description of drawings

> FIG. 1 is a diagrammatic lateral view of a centrifugal fractionation apparatus according to the invention;
> FIG. 2 is broken away view slightly from above of an apparatus corresponding to that shown in FIG. 1;
> FIG. 3 is a high-perspective view slightly from above of a multi-sample processing apparatus according to the invention;
> FIG. 4 is a high-perspective view slightly from below of the apparatus shown in FIG. 3;
> FIG. 5 is a broken away top view of an apparatus according to the invention; and
> FIG. 6 is an enlarged view of the encircled area 6 in FIG. 5 where a fractionated sample has been divided into compartments.

## Detailed description of exemplary embodiments

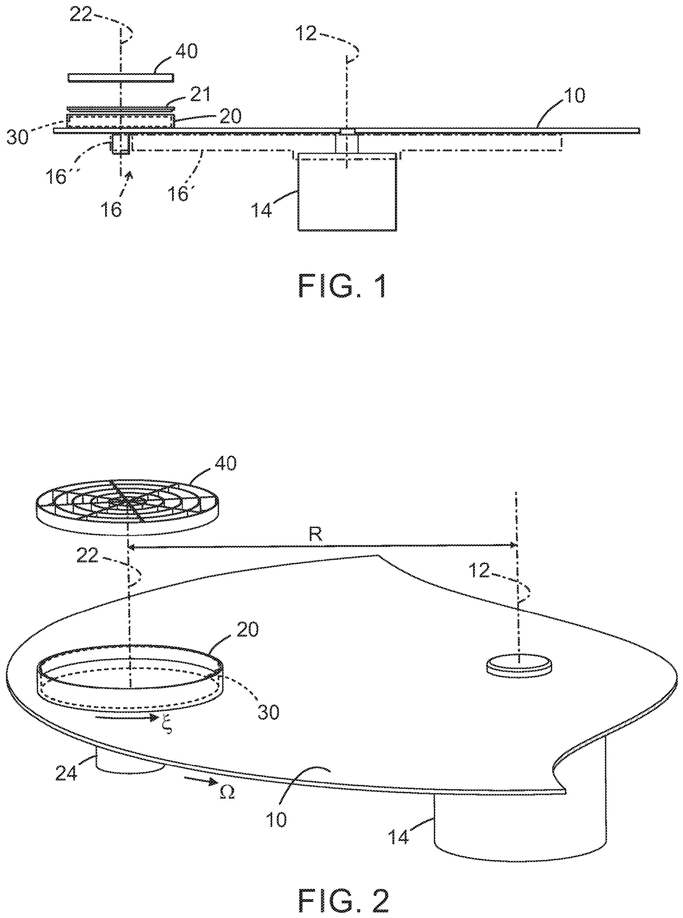

The fractionation apparatus shown in FIGS. 1 and 2, has a disk 10 supported for rotation about a primary axis 12. A relatively flat container or hollow cylinder 20 is supported at a peripheral portion of the disk 10 for rotation about a secondary axis 22 at a distance R from the primary axis 12. In the example shown in FIG. 2, disk 10 and container 20 are rotated by respective electric motors 14 and 24.

Other arrangements are likewise applicable, such as various gear or belt transmissions to rotate disk 10 and container 20 synchronously at different speeds by a single electric motor 14.

In such case, as indicated in phantom in FIG. 1, a gear transmission 16 may then include a stationary gear 16' in engagement with a gear 16" for rotating the container 20 synchronously with the disk 20 that is rotated by the electric motor 14.

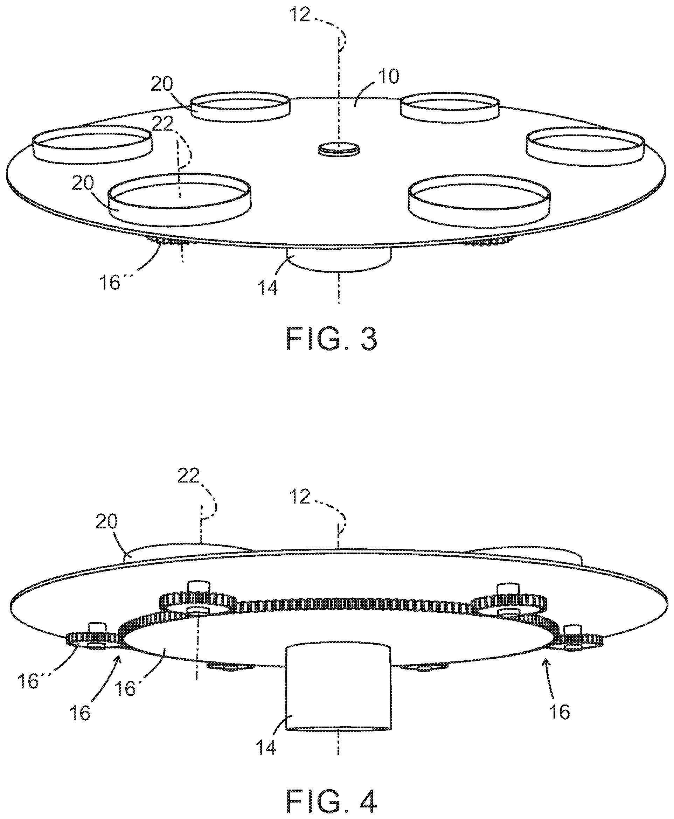

As shown in FIGS. 3 and 4, a plurality of samples may also be processed simultaneously by arranging a plurality of containers 20 peripherally distributed around the primary axis 12 where each container is rotated by gear transmissions 16 having a common stationary gear 16'.

A fractionation apparatus according to the invention may be operated as follows.

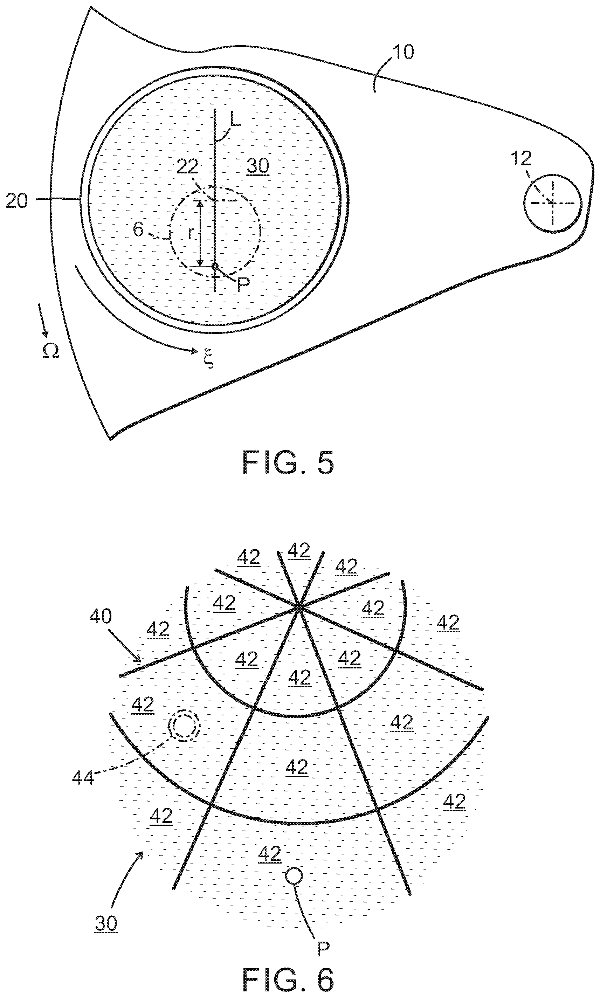

The cylindrical container 20 is filled with a liquid sample or suspension 30 having particles to be detected and identified. The container 20 is closed by a lid 21. Container 20 is then rotated at a speed $\xi$ different from and higher than a speed $\Omega$ of the disk 20. Due to the rotation about the primary axis 12, particles, such as bacteria and blood cells in blood, with a different density than the liquid will sediment radially outwards relative to the liquid. By the fact that the liquid does not have the very same rotation as the cylinder has around the primary axis 12, the particle P will sometimes be between the primary and the secondary axes and sometimes be located radially outside both axes. This causes the particle to alternately move from and towards the secondary axis. As previously mentioned, the sedimentation of particles of a certain size will at some point be counteracted by the rotation of the secondary axis so that the particle lies still relative to the primary axis. Each particle size has its equilibrium position when settled in the sample. Calculations have shown that the particle will search for the equilibrium position regardless of where in the sample it starts. The equilibrium position depends, among other things, on the particle's sedimentation rate, which in turn is affected by the size. The larger the particle, the farther from the secondary axis it ends up, and supposedly always on a straight line, L from the secondary axis as illustrated in FIG. 5.

When the above described centrifugal operation is completed, the particles may be spotted and identified.

If needed for certain applications, the particles can be spotted during the centrifugation process for example by a camera rotating together with the sample (not shown).

In order to easily classify the particles to be spotted, a sedimentation rate filter 40 is inserted into the settled sample 30 in the container 20. In the embodiment shown, the filter is shaped as a framework 40 defining axially open compartments 42 (FIG. 6) which framework 40 divides the settled sample 30 into radially and tangentially separated regions. When spotting a particle thus locked in a certain region, the information of the location of the region may facilitate identification of the particle for example by matching the location with locations in a table disclosing locations for already identified particles that have been subjected to an identical centrifugal operation. These tabulated locations may have been calculated numerically or determined by operating the fractionation apparatus on known particles under same conditions as those for the particles to be identified.

As an alternative to the framework 40, it would also be possible to draw specimens of the settled sample through valves arranged in a pattern substantially corresponding to the compartments 42, for example in the bottom of the container 20, as diagrammatically indicated in FIG. 6 that shows a single valve 44 in phantom.

Examples of calculating the behavior of particles subject to operation of a fractionation apparatus according to the invention are described in the following.

Let the difference in rotational speed between the rotation $\Omega$ about the primary axis and the rotation $\xi$ of the liquid about the secondary axis, i.e. the axis of the cylinder, be $\omega$. During the time $t = \pi / \omega$, the centrifugal forces will move the particle away from the cylinder axis and for the same time they will move the particle towards the cylinder axis. In addition, the particle follows the liquid around the cylinder axis. The farther from the centre of the cylinder the centrifugal forces have brought the particle, the faster it is brought back by the rotation of the liquid. This will create a spiralling motion towards the equilibrium position.

An $x$- $y$ coordinate system may be introduced (not shown) having its centre in the centre of the cylinder and which rotates with the rotational speed $\Omega$ of the cylinder around the main centre (located at the distance R far outside the cylinder). In this coordinate system, the centrifugal force is always directed in the y-direction.

Particles will end up at a distance from the cylinder centre which is approximately proportional to the sedimentation velocity. A double radius particle ends up nearly four times further away from the cylinder centre. A different starting position of a particle does not seem to affect the particle's end position.

It is possible to show that

$$r = \frac{2}{9} \frac{\Delta\rho}{\rho} \frac{a^2}{v} \frac{R\Omega^2}{\omega}$$

where

$a$ is the particle radius
$R$ is the distance between the two axes
$\Delta\rho$ is the difference in mass density between the particle and the fluid sample
$\rho$ is the mass density of the fluid sample, and
$v$ is the dynamic viscosity of the fluid.
$r$ is proportional to the square of particle radius. It should therefore be possible to separate particles with almost the same radius a.

(056) The centrifugal force $R\Omega^2$ must be large enough compared to the relative rotation $\omega$ for the particle to end up far enough from the cylinder centre. At the same time, $\omega$ must not be too small because then the process takes too long.

(057) The foregoing detailed description is given primarily for clearness of understanding and no unnecessary limitations are to be understood therefrom. Modifications will become obvious to those skilled in the art upon reading this disclosure and may be made without departing from the scope of the appended claims.

**Claims**

1. A method of separating particles (P) having different sedimentation velocities in a fluid sample (30) through centrifugal sedimentation, comprising:

a) enclosing the sample (30); and
b) rotating the sample about a primary axis (12) outside the sample at a first rotational speed ($\Omega$), and about a secondary axis (22) located in a center of the sample at a second rotational speed ($\xi$), for subjecting the sample to a varying centrifugal field until each particle has settled at a position which depends on the sedimentation velocity of the particle.

2. The method of claim 1, further comprising

c) spotting the particles (P) in the settled fractions in the sample;
d) measuring a distance (r) of each particle from the secondary axis (22); and
e) identifying the particles by matching said distance with distances obtained by calculation for known particles theoretically subjected to the method.

3. The method of claim 1, further comprising

c) spotting the particles (P) in the settled fractions in the sample;
d) measuring a distance (r) of each particle from the secondary axis (22); and
f) identifying the particles by matching said distance with distances obtained by performing the above steps a-d for known particles.

4. The method of any of the preceding claims, further comprising blocking different regions (42) of the sample from mixing with each other.

5. The method of claim 4, comprising inserting a framework (40) of axially open compartments (42) into the sample (30) having the settled fractions, for dividing the sample into said regions (42) defined by the compartments.

6. The method of any of claims 1-3, comprising sampling particles from different regions (42) in separate samples.

7. The method of any of the preceding claims, wherein the second rotational speed ($\xi$) is higher than the first rotational speed ($\Omega$).

8. The method of any of the preceding claims, wherein the sample is a blood sample.

9. An apparatus for performing the method of claim 1, wherein

a cylindrical container (20) for enclosing the sample (10);
a first rotator (10, 14) for rotating the container (20) about the primary axis (12); and
a second rotator (16; 24) for rotating the container (20) about the secondary axis (22),
wherein the first rotator comprises a disk (10) supported for rotation about the primary axis (12) and an electric motor (14) for rotating the disk (10), and the second rotator comprises a gear or belt transmission (16) for rotating the cylindrical container (20) supported at the disk about the secondary axis (22) by the electric motor (14).

10. The apparatus of claim 9, wherein the second rotator comprises a stationary gear (16') concentric with the primary axis and in gear engagement with a gear (16") for rotating the cylindrical container (20) supported at the disk about the secondary axis (22).

11. The apparatus of any of claims 9-10, comprising a plurality of said container (20) peripherally distributed around the primary axis (12).

12. The apparatus of any of claims 9-11, comprising means (40) for dividing the sample in separated regions (42).

13. The apparatus of claim 12, wherein the means for dividing the sample (30) comprises a framework (40) of axially open compartments (42) to be inserted into the settled sample in the container (20).

**Patentansprüche**

1. Verfahren zur Trennung von Partikeln (P) mit unterschiedlichen Sedimentationsgeschwindigkeiten in einer Flüssigkeitsprobe (30) durch zentrifugale Sedimentation, umfassend:

a) Einschließen der Probe (30); und
b) Rotieren der Probe um eine Primärachse (12) außerhalb der Probe mit einer ersten Rotationsgeschwindigkeit ($\Omega$) und um eine Sekundärachse (22) im Zentrum der Probe mit einer zweiten Rotationsgeschwindigkeit ($\xi$), um die Probe einem variierenden Zentrifugalfeld auszusetzen, bis sich jedes Partikel an einer von seiner Sedimentationsgeschwindigkeit abhängigen Position abgesetzt hat.

2. Verfahren nach Anspruch 1, umfassend:

c) Auffinden der Partikel (P) in den abgesetzten Fraktionen der Probe;
d) Messen des Abstands (r) jedes Partikels von der Sekundärachse (22); und

e) Identifizieren der Partikel durch Vergleichen dieses Abstands mit berechneten Abständen für bekannte, theoretisch dem Verfahren unterzogene Partikel.

3. Verfahren nach Anspruch 1, umfassend:

c) Auffinden der Partikel (P) in den abgesetzten Fraktionen der Probe;
d) Messen des Abstands (r) jedes Partikels von der Sekundärachse (22); und
f) Identifizieren der Partikel durch Vergleichen dieses Abstands mit den Abständen, die durch die Durchführung der obigen Schritte a-d für bekannte Partikel ermittelt wurden.

4. Verfahren nach einem der vorhergehenden Ansprüche, umfassend das Verhindern der Vermischung verschiedener Bereiche (42) der Probe.

5. Verfahren nach Anspruch 4, umfassend das Einsetzen eines Gerüsts (40) aus axial offenen Kammern (42) in die Probe (30) mit den abgesetzten Fraktionen, um die Probe in die durch die Kammern definierten Bereiche (42) zu unterteilen.

6. Verfahren nach einem der Ansprüche 1-3, umfassend die Entnahme von Partikeln aus verschiedenen Bereichen (42) in getrennten Proben.

7. Verfahren nach einem der vorhergehenden Ansprüche, wobei die zweite Rotationsgeschwindigkeit ($\xi$) höher ist als die erste Rotationsgeschwindigkeit ($\Omega$).

8. Verfahren nach einem der vorhergehenden Ansprüche, wobei die Probe eine Blutprobe ist.

9. Vorrichtung zur Durchführung des Verfahrens nach Anspruch 1, mit einem zylindrischen Behälter (20) zum Einschließen der Probe (10);

einem ersten Rotator (10, 14) zum Drehen des Behälters (20) um die Primärachse (12); und einem zweiten Rotator (16; 24) zum Drehen des Behälters (20) um die Sekundärachse (22). Der erste Rotator umfasst eine um die Primärachse (12) drehbar gelagerte Scheibe (10) und einen Elektromotor (14) zum Drehen der Scheibe (10). Der zweite Rotator umfasst ein Zahnrad- oder Riemengetriebe (16) zum Drehen des an der Scheibe gelagerten zylindrischen Behälters (20) um die Sekundärachse (22) durch den Elektromotor (14).

10. Vorrichtung nach Anspruch 9, wobei der zweite Rotator ein stationäres Zahnrad (16') umfasst, das konzentrisch zur Primärachse angeordnet ist und mit einem Zahnrad (16") in Eingriff steht, um den an der Scheibe gelagerten zylindrischen Behälter (20) um die Sekundärachse (22) zu drehen.

11. Vorrichtung nach einem der Ansprüche 9-10, umfassend eine Vielzahl von Behältern (20), die peripher um die Primärachse (12) verteilt sind.

12. Vorrichtung nach einem der Ansprüche 9-11, umfassend Mittel (40) zum Aufteilen der Probe in getrennte Bereiche (42).

13. Vorrichtung nach Anspruch 12, wobei die Mittel zum Aufteilen der Probe (30) ein Gerüst (40) mit axial offenen Kammern (42) umfassen, die in die im Behälter (20) abgesetzte Probe eingesetzt werden.

**Revendications**

1. Procédé de séparation de particules (P) présentant différentes vitesses de sédimentation dans un échantillon fluide (30) par sédimentation centrifuge, comprenant:

a) l'encapsulation de l'échantillon (30); et
b) la rotation de l'échantillon autour d'un axe primaire (12) extérieur à une première vitesse de rotation ($\Omega$), et autour d'un axe secondaire (22) situé au centre de l'échantillon à une seconde vitesse de rotation ($\xi$), afin de soumettre l'échantillon à un champ centrifuge variable jusqu'à ce que chaque particule se dépose à une position dépendant de sa vitesse de sédimentation.

2. Procédé selon la revendication 1, comprenant en outre:

c) le repérage des particules (P) dans les fractions sédimentées de l'échantillon;
d) la mesure de la distance (r) de chaque particule par rapport à l'axe secondaire (22); et
e) l'identification des particules en comparant cette distance aux distances calculées pour des particules connues soumises théoriquement à la méthode.

3. Procédé selon la revendication 1, comprenant en outre:

c) le repérage des particules (P) dans les fractions déposées de l'échantillon;
d) la mesure de la distance (r) de chaque particule par rapport à l'axe secondaire (22); et
f) l'identification des particules en comparant cette distance aux distances obtenues en effectuant les étapes a à d ci-dessus pour des particules connues.

4.  Procédé selon l'une quelconque des revendications précédentes, comprenant en outre l'empêchement du mélange de différentes régions (42) de l'échantillon.

5.  Procédé selon la revendication 4, comprenant l'insertion d'une structure (40) de compartiments (42) ouverts axialement dans l'échantillon (30) contenant les fractions déposées, afin de diviser l'échantillon en régions (42) définies par les compartiments.

6.  Procédé selon l'une quelconque des revendications 1 à 3, comprenant l'échantillonnage de particules provenant de différentes régions (42) dans des échantillons distincts.

7.  Procédé selon l'une quelconque des revendications précédentes, dans lequel la seconde vitesse de rotation ($\xi$) est supérieure à la première vitesse de rotation ($\Omega$).

8.  Procédé selon l'une quelconque des revendications précédentes, dans lequel l'échantillon est un échantillon de sang.

9.  Appareil pour la mise en œuvre du procédé selon la revendication 1, comprenant:

    un récipient cylindrique (20) contenant l'échantillon (10);
    un premier rotateur (10, 14) faisant tourner le récipient (20) autour de l'axe principal (12); et
    un second rotateur (16 ; 24) faisant tourner le récipient (20) autour de l'axe secondaire (22). Le premier rotateur comprend un disque (10) supporté en rotation autour de l'axe principal (12) et un moteur électrique (14) assurant la rotation du disque (10), et le second rotateur comprend une transmission par engrenage ou courroie (16) assurant la rotation du récipient cylindrique (20) supporté par le disque autour de l'axe secondaire (22) par le moteur électrique (14).

10. Appareil selon la revendication 9, dans lequel le second rotateur comprend un engrenage fixe (16') concentrique à l'axe principal et en prise avec un engrenage (16") pour faire tourner le récipient cylindrique (20) supporté par le disque autour de l'axe secondaire (22).

11. Appareil selon l'une quelconque des revendications 9 et 10, comprenant plusieurs récipients (20) répartis périphériquement autour de l'axe principal (12).

12. Appareil selon l'une quelconque des revendications 9 à 11, comprenant un moyen (40) pour diviser l'échantillon en zones séparées (42).

13. Appareil selon la revendication 12, dans lequel le moyen de division de l'échantillon (30) comprend un cadre (40) composé de compartiments (42) ouverts axialement, destinés à être insérés dans l'échantillon déposé dans le récipient (20).

FIG. 1

FIG. 2

FIG. 3

FIG. 4

FIG. 5

FIG. 6

**EP 4 017 643 B1**

REFERENCES CITED IN THE DESCRIPTION

**Patent documents cited in the description**

- WO 2019091880 A1
- WO 2019091650 A1
- WO 2011081531 A1